# EUROPÄISCHE PATENTSCHRIFT

(11) **EP 2 357 011 B1**
(45) Veröffentlichungstag und Bekanntmachung des Hinweises auf die Patenterteilung: **22.08.2012**
(21) Anmeldenummer: 10014189.4
(22) Anmeldetag: 02.11.2010
(51) Int. Cl.: A61L 9/12

(54) **Verdampfer mit einem Behälter und einem aus dem Behälter ragenden Docht sowie einer Kappe mit Führung zur Abdeckung unterschiedlicher Dochtlängen**
Evaporator with a container and a wick protruding out of the container and a cap with channel for covering different wick lengths
Evaporateur doté d'un récipient et d'une mèche sortant du récipient ainsi que d'une cape dotée d'un guidage pour recouvrir les différentes longueurs de mèche

(30) Priorität: 13.01.2010 DE 102010004599; 04.05.2010 DE 202010006425 U
(43) Veröffentlichungstag der Anmeldung: 17.08.2011
(73) Patentinhaber: Pont Packaging GmbH, 41066 Mönchengladbach (DE)
(72) Erfinder: Salmann, Frank, 41239 Mönchengladbach (DE)
(74) Vertreter: Castell, Klaus

(56) Entgegenhaltungen:
- EP-A1- 1 031 446
- EP-A1- 1 839 684
- DE-U1-202010 006 425
- US-A1- 2006 016 904
- US-A1- 2007 119 963

## Beschreibung

Die Erfindung betrifft einen Verdampfer mit einem Behälter, der einen Reservoirbereich aufweist, und einem aus dem Behälter ragenden Docht sowie einer Kappe, um den aus dem Behälter ragenden Docht abzudecken, wobei die Kappe Löcher und eine Führung mit einer Dochtabdeckung aufweist, wobei die Führung es erlaubt, mit der Kappe unterschiedliche Dochtlängen abzudecken. Derartige Verdampfer werden dazu verwendet, einen Geruchsstoff an Räume abzugeben. Hierzu wird der Behälter mit einem flüssigen Medium befüllt, das im Docht aufsteigt. Vor der Nutzung des Verdampfers ist der Docht mit einer Kappe luftdicht abgeschlossen, so dass eine Verdampfung ausgeschlossen ist. Wenn der Verdampfer in einem Raum eingesetzt wird, wird zunächst die Kappe entfernt, so dass im Docht aufsteigende Flüssigkeit den Behälter verlassen kann und kontinuierlich an die Umgebung abgegeben wird. Durch Wiederaufschrauben der Kappe kann die Abgabe von Dämpfen unterbunden werden. Sofern die Kappe lange genug geöffnet bleibt, verdampft die gesamte Flüssigkeit aus einem Flüssigkeitsreservoir an die Umgebung.

Ein gattungsgemäßer Verdampfer ist aus der US 20060016904 A1 bekannt. Dieser mehrteilige Verdampfer sieht eine Führung innerhalb eines Flüssigkeitsreservoirs vor. Er ist kompakt im Aufbau aber teuer in der Herstellung, da unterschiedliche Teile gefertigt und zusammengebaut werden müssen.

Der vorliegenden Erfindung liegt die Aufgabe zugrunde, derartige Verdampfer weiterzuentwickeln, um sie günstiger herstellen zu können, ohne dass die Funktion dadurch beeinträchtigt wird. Diese Aufgabe wird mit einem Verdampfer mit den Merkmalen des Patentanspruchs 1 gelöst.

Dadurch dass der Behälter ein Außengewinde aufweist, das einstückig mit dem Reservoirbereich ausgebildet ist, wird die Herstellung derartiger Massenprodukte deutlich vereinfacht, ohne dass auf eine vorteilhafte Funktion des Verdampfers verzichtet werden muss.

Durch die Möglichkeit, unterschiedliche Dochtlängen mittels der Kappe abzudecken, erweitert sich der Einsatzbereich derartiger Verdampfer, die nun auch eine dosierte Menge an Flüssigkeit in dampfförmiger Form abgeben können. Die spezielle Ausführungsform einer Kappe mit einer derartigen Führung ermöglicht es ohne weitere Teile direkt mit der notwendigen Kappe auch eine Dosierfunktion zu erzielen.

Die vorzugsweise als Spritzgussteil hergestellte Kappe ist dabei so ausgeformt, dass sie mittels der über die Führung verschiebbaren Kappe unterschiedliche Dochtlängen abdeckt bzw. freigibt und dadurch es ermöglicht, die pro Zeiteinheit abgegebene Dampfmenge zu variieren. Hierbei ist von besonderem Vorteil, dass die Dosierfunktion mit der notwendigen Kappe erzielt wird und somit die gleiche Kappe einerseits ein luftdichtes Verschließen eines Reservoirbereichs erlaubt und andererseits durch die spezielle Ausbildung der Formgebung der Kappe auch eine Dosierung der abgegebenen Dampfmenge ermöglicht.

Dies wird auf einfache Art und Weise dadurch erzielt, dass die Führung eine laterale Bewegung zwischen Behälter und Kappe ermöglicht. Bereits ein leichtes Anheben der Kappe relativ zum Behälter gibt einen kleinen Spalt des Dochtes frei, um geringe Mengen an Flüssigkeit verdampfen zu lassen. Eine stärkere laterale Bewegung zwischen Behälter und Kappe führt zu einer geringeren Abdeckung des Dochtes und damit zu einem größeren Verdampferspalt, der eine stärkere Verdampfung pro Zeiteinheit ermöglicht.

Da die Führung ein Gewinde aufweist, kann durch Zusammenwirken mit einer entsprechenden Ausformung am Behälter mittels einer Drehbewegung der Kappe relativ zum Behälter eine laterale Bewegung zwischen Behälter und Kappe erzielt werden, die es ermöglicht, die Abdeckung des Dochtes zu variieren.

Ein besonders kompakter Aufbau eines Verdampfers wird dadurch erzielt, dass der Behälter einen Reservoirbereich aufweist, wobei der Durchmesser der Führung kleiner ist als der des Reservoirbereichs. Dies ermöglicht es, einen besonders großen Reservoirbereich mit einem großen Durchmesser vorzusehen, während radial innerhalb des Reservoirbereichs eine Führung vorgesehen ist, die es erlaubt, die Kappe relativ zum Behälter zu bewegen.

Eine besonders gute Ausnutzung des zur Verfügung stehenden Raumes bei minimalem Materialeinsatz wird dadurch erzielt, dass der Behälter bis in die Führung hinein befüllt ist.

Damit leicht erkannt werden kann, wie die Kappe unterschiedliche Dochtlängen abdecken kann, weist die Kappe Löcher auf, durch die hindurch der Docht sichtbar ist. Diese Löcher können einerseits dazu dienen, Dämpfe an die Umgebung abzugeben. Sie haben jedoch auch den Vorteil, dass erkannt werden kann, wie viel Docht abgedeckt ist und ob der Docht noch mit Flüssigkeit getränkt ist. Bei einem leeren Behälter ist in der Regel an der Farbgebung des Dochtes leicht zu erkennen, dass der Docht ausgetrocknet ist und der Behälter neu befüllt oder ausgetauscht werden muss.

Während der Behälter in der Regel durchsichtig ist, um die Befüllung leicht von außen wahrnehmen zu können, erlaubt der durch die Löcher sichtbare Docht den Behälter auch undurchsichtig auszuführen.

Funktion und Design führen zu einer Ausführungsvariante, bei der die Kappe einen Außendurchmesser aufweist, der dem Durchmesser des Reservoirbereichs entspricht. Dadurch kann ein im Wesentlichen kugelförmiger Verdampfer hergestellt werden, der minimalen Materialeinsatz mit einem maximalen inneren Volumen verbindet.

Eine spezielle Ausführungsform sieht vor, dass die Kappe einen Luftspalt zwischen Außendurchmesser und Führung aufweist.

Weiterhin wird vorgeschlagen, dass die Kappe eine radial innerhalb der Führung liegende Dochtabdeckung aufweist. Dadurch wird eine ergonomische Form des Verdampfers erzielt.

Eine besonders vorteilhafte Behältergröße ergibt sich, wenn der Behälter so ausgeführt ist, dass er mit etwa 50 bis 250 ml Flüssigkeit befüllbar ist. Daher wird vorgeschlagen, dass der Behälter etwa 50 bis 250 ml Flüssigkeit aufweist.

Eine besonders vorteilhafte Ausführungsform eines erfindungsgemäßen Verdampfers ist in der Zeichnung dargestellt und wird im Folgenden näher erläutert. Es zeigt
- Figur 1: einen Schnitt durch einen Verdampfer mit aufgeschraubter Kappe,
- Figur 2: einen Schnitt durch einen Verdampfer mit abgeschraubter Kappe,

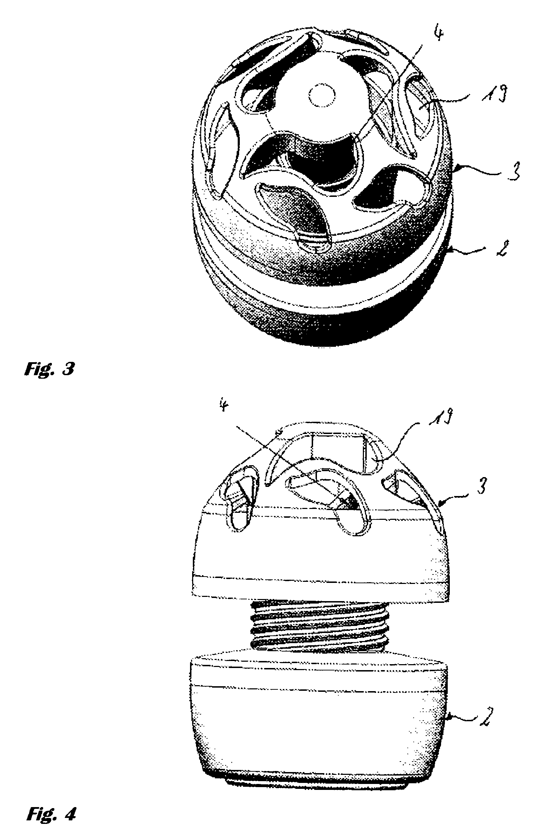

- Figur 3: eine perspektivische Aufsicht auf den Verdampfer nach Figur 2,
- Figur 4: eine Seitenansicht des in Figur 2 dargestellten Verdampfers,
- Figur 5: einen Schnitt durch eine alternative Ausführungsform eines Verdampfers mit aufgeschraubter Kappe,
- Figur 6: einen Schnitt durch den in Figur 5 gezeigten Verdampfer mit abgeschraubter Kappe,
- Figur 7: eine perspektivische Aufsicht auf den Verdampfer nach Figur 5,
- Figur 8: eine Seitenansicht des in Figur 5 dargestellten Verdampfers
- Figur 9: einen Verdampfer mit eingezogenem Boden und
- Figur 10: eine perspektivische Ansicht des Verdampfers nach Figur 9.

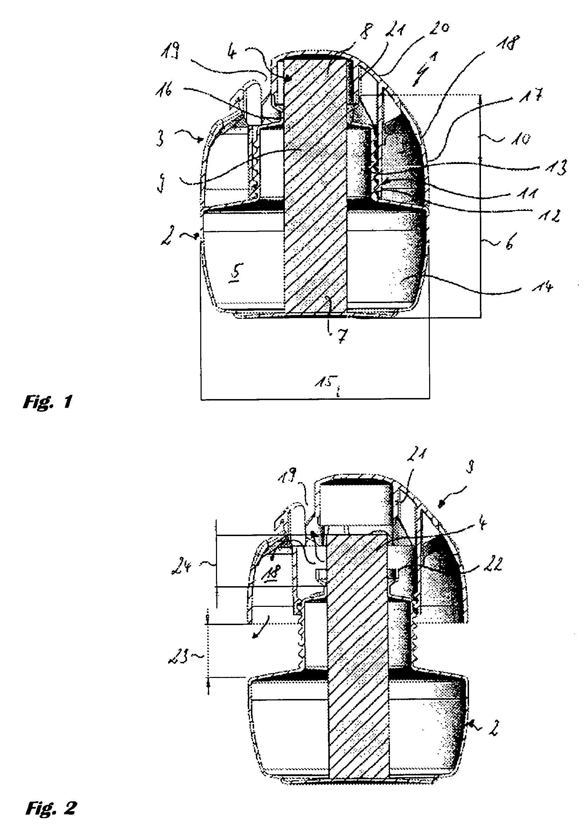

Der in Figur 1 gezeigte Verdampfer 1 besteht im Wesentlichen aus einem Behälter 2, einer Kappe 3 und einem Docht 4. Im Behälter 2 ist eine Flüssigkeit 5, die den Behälter 2 bis zu einer Füllhöhe 6 befüllt. Das untere Ende 7 des Dochtes 4 ragt somit in die Flüssigkeit 5, während das obere Ende 8 des Dochtes 4 aus der Flüssigkeit und aus dem Behälter 2 hervorragt. Ein unteres Teilstück 7 des Dochtes 4 erstreckt sich über die Länge 6 in die Flüssigkeit 5 hinein. Ein oberes Teilstück 8 des Dochtes 4 ragt aus dem Behälter 2 hervor und ein dazwischen liegendes Teilstück 9 des Dochtes 4 liegt im Bereich 10 innerhalb des Behälters 2, aber außerhalb der Flüssigkeit 5.

Die Kappe 3 hat eine Führung 11, die als Innengewinde 12 ausgebildet ist und mit einem Außengewinde 13 am Behälter 2 zusammenwirkt.

Der Behälter 2 hat an seinem unteren Ende einen Reservoirbereich 14, dessen Durchmesser 15 größer ist als der Durchmesser der Führung 11 mit ihrem Innengewinde 12 und ihrem Außengewinde 13. Wenn der Behälter 1 ausgeliefert wird, ist er voll befüllt. In diesem Zustand ist der Behälter 2 bis in die Führung 11 hinein befüllt.

Der Behälter 2 hat oberhalb der Führung 11 einen eingeschnittenen Kragen 16, dessen Innendurchmesser etwa dem Außendurchmesser des Dochtes 4 entspricht. In diesem Kragen 16 wird der Docht gehalten, dessen oberes Ende 8 aus dem Kragen 16 und aus dem Behälter 2 hervorschaut.

Die Kappe 3 hat einen Außendurchmesser 17, der dem Außendurchmesser 15 des Behälters 2 entspricht. Zwischen diesem Außendurchmesser 17 und der Führung 11 ist ein Luftspalt 18 in der Kappe 3 vorgesehen. Am oberen Ende der Kappe 3 sind Löcher 19 vorgesehen, die die äußere Schale 20 der Kappe 3 durchbrechen.

Bei fest aufgeschraubter Kappe 3 auf den Behälter 2 wirkt ein umlaufender Flansch 21 mit dem oberen Ende des Behälters 2 derart zusammen, dass der Behälter 2 mit dem vorstehenden Docht 4 luftdicht abgeschlossen wird.

Wenn die Kappe 3 relativ zum Behälter 2 gedreht wird, hebt sich die Kappe 3 durch eine laterale Bewegung relativ zum Behälter 2 ab und es öffnet sich ein Spalt zwischen dem Flansch 21 und dem oberen Ende 22 des Behälters 2. Durch diesen Spalt gelangen Dämpfe vom Docht 4 zu den Löchern 19 und zum Luftraum 18, wodurch diese Dämpfe aus dem Verdampfer entweichen können.

Wenn die Kappe 3 über die Länge 23 abgehoben wird, wird ein Teilstück des Dochtes 4 auf der Länge 24 von der Kappe 3 freigegeben, um Dämpfe abzugeben. Die Figur 2 zeigt deutlich, dass mit dem umlaufenden Flansch 21 in der Kappe 3 durch Drehung der Kappe 3 relativ zum Behälter 2 unterschiedliche Längen des Dochtes 4 abgedeckt bzw. freigelegt werden können. Dies ermöglicht es, durch einfache Drehung der Kappe 3 relativ zum Behälter 2 die Verdampfungsmenge am Docht 4 zu regulieren.

Der Anwender erkennt die Stärke der eingestellten Verdampfungsleistung am Abstand 23 zwischen dem unteren Ende der Kappe 3 und dem Behälter 2. Außerdem kann der Anwender durch die Löcher 19 bei aufgeschraubter Kappe 3 bis zum Docht 4 sehen und dadurch erkennen, wie viel des Dochtbereiches frei zugänglich sind und dadurch als Verdampfungsbereich wirken.

Die Kappe 3 kann jederzeit auch wieder vollständig oder teilweise auf den Behälter 2 aufgeschraubt werden, damit der umlaufende Flansch 21 wieder einen größeren Bereich des Dochtes 4 als Dochtabdeckung abdeckt oder sogar den Behälter 2 verschließt.

Je nach Ausbildung der Führung 11 bzw. der Gewinde 12 und 13 kann die Kappe 3 unverlierbar mit dem Behälter 2 verbunden sein oder auch vollständig abnehmbar sein. Sofern eine Nachbefüllung des Behälters 2 vorgesehen ist, wird die Kappe vollständig abnehmbar ausgebildet. Sofern der Verdampfer als Einwegeinrichtung nicht zur Nachbefüllung vorgesehen ist, verhindert ein Anschlag (nicht gezeigt) an der Führung 11 das vollständige Abschrauben der Kappe 3 vom Behälter 2.

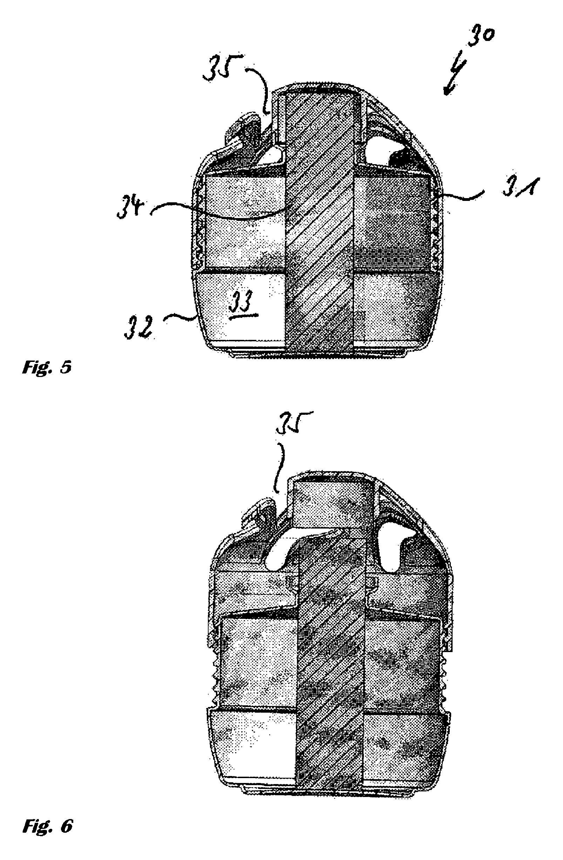

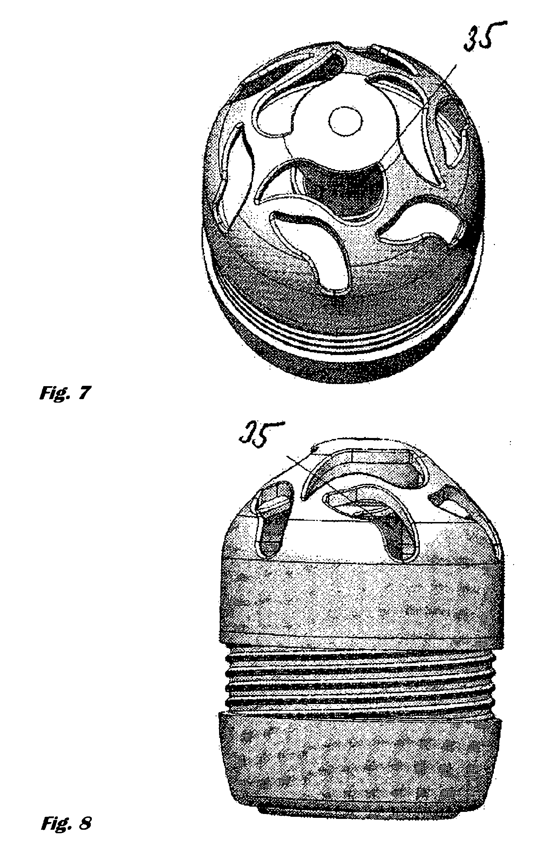

Bei dem in den Figuren 5 bis 8 gezeigten Verdampfer 30 ist das innere Gewinde 31 radial nach Außen gesetzt. Dadurch kann der Verdampfer 30 in Behälter 32 mehr Flüssigkeit 33 aufnehmen. Die Strömung vom Docht 34 durch die Löcher 35 wird dadurch kaum beeinträchtigt. In der Praxis konnte hierdurch sogar die pro Zeiteinheit verdampfte Menge gesteigert werden.

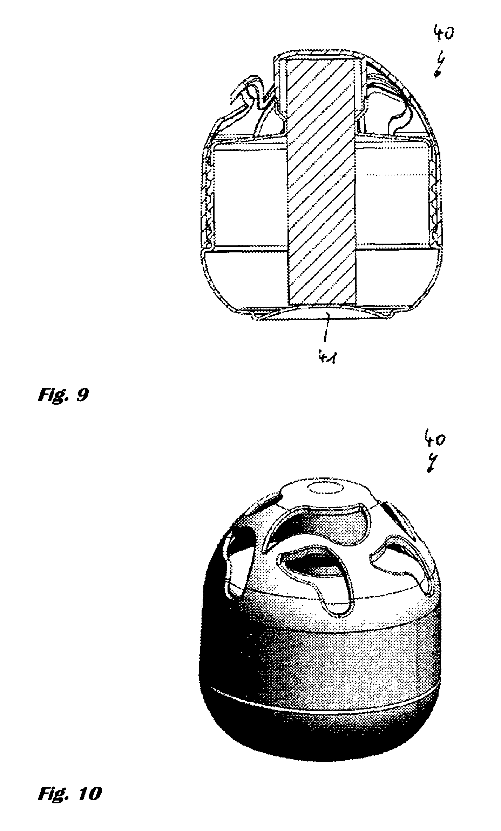

Der Verdampfer 40, der in den Figuren 9 und 10 gezeigt ist, hat einen eingezogenen Boden 41. Im Übrigen ist der Aufbau fast identisch zu den zuvor beschriebenen Verdampfern.

## Patentansprüche

1. Verdampfer (1) mit einem Behälter (2), der einen Reservoirbereich aufweist, und einem aus dem Behälter (2) ragenden Docht (4) sowie einer Kappe (3), um den aus dem Behälter 2 ragenden Docht (4) abzudecken, wobei die Kappe (3) Löcher (19) aufweist, durch die hindurch der Docht (4) sichtbar ist, und wobei die Kappe (3) eine als Innengewinde (12) ausgebildete Führung (11) aufweist, die eine Laterale Bewegung zwischen Behälter (2) und Kappe (3) ermöglicht, und die es erlaubt mit der Kappe (3) unterschiedliche Dochtlängen abzudecken
**dadurch gekennzeichnet,**
**dass** der Behälter (2) ein zusammen mit dem Reservoirbereich einstückig ausgebildetes Aussengewinde (13) aufweist, das mit dem Innengewinde (12) der Führung (11) zusammenwirkt.

2. Verdampfer nach Anspruch 1, ***dadurch gekennzeichnet, dass*** der Behälter (2) oberhalb der Führung (11) einen eingeschnittenen Kragen (16) hat, dessen Innendurchmesser etwa dem Außendurchmesser des Dochtes (4) entspricht.

3. Verdampfer nach einem der vorhergehenden Ansprüche, ***dadurch gekennzeichnet, dass*** der Durchmesser der Führung (11) kleiner ist als der des Reservoirbereichs (14).

4. Verdampfer nach einem der vorhergehenden Ansprüche, ***dadurch gekennzeichnet, dass*** der Behälter (2) bis in die Führung (11) hinein befüllt ist.

5. Verdampfer nach einem der vorhergehenden Ansprüche, ***Dadurch gekennzeichnet, dass*** die Kappe (3) einen Außendurchmesser (17) aufweist, der dem Durchmesser (15) des Behälters (2) entspricht.

6. Verdampfer nach einem der vorhergehenden Ansprüche, ***Dadurch gekennzeichnet, dass*** die Kappe (3) einen Luftspalt (18) zwischen Außendurchmesser (17) und Führung (11) aufweist.

7. Verdampfer nach einem der vorhergehenden Ansprüche, ***dadurch gekennzeichnet, dass*** die Dochtabdeckung (21) radial innerhalb der Führung (11) liegt.

8. Verdampfer nach einem der vorhergehenden Ansprüche, ***dadurch gekennzeichnet, dass*** der Behälter (2) etwa 50 bis 250 ml Flüssigkeit aufweist.

## Claims

1. An evaporator (1) with a container (2) and a wick (4) protruding out of the container (2) as well as a cap (3) for covering the wick (4) protruding out of the container (2), wherein the cap (3) comprises holes (19) through which the wick (4) is visible, and wherein the cap (3) comprises a channel (11) formed as an internal thread (12) which permits lateral movement between the container (2) and the cap (3) and which allows different wick lengths to be covered by the cap (3),
***characterised in that***
the container (2) comprises an external thread (13) formed in one piece with the reservoir area, which external thread cooperates with the internal thread (12) of channel (11).

2. Evaporator according to claim 1, ***characterised in that*** the container (2) has a collar (16) which is cut-in above the channel (11) and the inner diameter of which roughly corresponds to the outer diameter of the wick (4).

3. Evaporator according to one the above claims, ***characterised in that*** the diameter of the channel (11) is smaller than that of the reservoir area (14).

4. Evaporator according to one the above claims, ***characterised in that*** the container (2) is filled as far as into the channel (11).

5. Evaporator according to one the above claims, ***characterised in that*** the cap (3) has an outer diameter (17) which corresponds to the diameter (15) of the container (2).

6. Evaporator according to one the above claims, ***characterised in that*** the cap (3) comprises an air gap (18) between the outer diameter (17) and the channel (11).

7. Evaporator according to one the above claims, ***characterised in that*** the wick cover (21) is arranged radially within the channel (11).

8. Evaporator according to one the above claims, ***characterised in that*** the container (2) holds approximately 50 to 250 ml liquid.

## Revendications

1. Evaporateur (1) avec un récipient (2) qui comporte une zone de réservoir et une mèche (4) saillant hors du récipient (2), ainsi qu'un capuchon (3) pour recouvrir la mèche (4) saillant hors du récipient (2), le capuchon (3) comportant des trous (19) à travers lesquels on peut voir la mèche (4) et le capuchon (3) comportant un guidage (11) conçu sous la forme d'un taraudage (12), qui permet un mouvement latéral entre le récipient (2) et le capuchon (3) et qui permet de recouvrir avec le capuchon (3) différentes longueurs de la mèche,
***caractérisé en ce que***
le récipient (2) comporte un filetage (13) conçu en monobloc avec la zone de réservoir, qui coopère avec le taraudage (12) du guidage (11).

2. Evaporateur selon la revendication 1, ***caractérisé en ce que*** le récipient (2) dispose au-dessus du guidage (11) d'un col entaillé (16) dont le diamètre intérieur correspond approximativement au diamètre extérieur de la mèche (4).

3. Evaporateur selon l'une quelconque des revendications précédentes, ***caractérisé en ce que*** le diamètre du guidage (11) est inférieur à la zone de réservoir (14).

4. Evaporateur selon l'une quelconque des revendications précédentes, **caractérisé en ce que** le récipient (2) est rempli jusqu'à l'intérieur du guidage (11).

5. Evaporateur selon l'une quelconque des revendications précédentes, ***caractérisé en ce que*** le capuchon (3) comporte le diamètre extérieur (17) qui correspond au diamètre (15) du récipient (2).

6. Evaporateur selon l'une quelconque des revendications précédentes, ***caractérisé en ce que*** le capuchon (3) comporte une fente d'aération (18) entre le diamètre extérieur (17) et le guidage (11).

7. Evaporateur selon l'une quelconque des revendications précédentes, ***caractérisé en ce que*** le recouvrement de la mèche (21) se situe en direction radiale, à l'intérieur du guidage (11).

8. Evaporateur selon l'une quelconque des revendications précédentes, ***caractérisé en ce que*** le récipient (2) comporte approximativement de 50 à 250 ml de liquide.
